# EUROPÄISCHE PATENTSCHRIFT

(11) **EP 1 196 336 B1**
(45) Veröffentlichungstag und Bekanntmachung des Hinweises auf die Patenterteilung: **12.03.2003**
(21) Anmeldenummer: 00953075.9
(22) Anmeldetag: 20.07.2000
(51) Int. Cl.: B65D 81/32, B65D 77/04

(54) **BLISTERVERPACKUNG**
BLISTER PACK
EMBALLAGE-COQUE

(30) Priorität: 24.07.1999 DE 29912954 U
(43) Veröffentlichungstag der Anmeldung: 17.04.2002
(73) Patentinhaber: Coripharm Medizinprodukte GmbH & Co. KG., 64807 Dieburg (DE)
(72) Erfinder: DINGELDEIN, Elvira, 63303 Dreieich (DE); WAHLIG, Helmut, 64287 Darmstadt (DE); SATTIG, Christoph, 64807 Dieburg (DE); WÜST, Edgar, 63110 Rodgau (DE)
(74) Vertreter: Helber, Friedrich G., Dipl.-Ing.
(86) Internationale Anmeldenummer: EP0006920
(87) Internationale Veröffentlichungsnummer: WO01007337

(56) Entgegenhaltungen:
- EP-A- 0 831 034
- US-A- 3 618 751
- US-A- 4 697 703
- US-A- 4 844 251

## Beschreibung

Die Erfindung betrifft eine Blister-Verpackungseinheit, in welcher wenigstens zwei unterschiedliche Komponenten unverlierbar enthalten sind, von denen wenigstens eine eine pulver- oder granulatförmige Konsistenz hat, die vor Gebrauch mit der bzw. den weiter abgepackten Komponente(n) in einer Misch- oder Applikations-vorrichtung zu einer gebrauchsfertigen Mischung aufbereitet werden sollen, wobei die Komponenten in entsprechend vorgeformten Aufnahmen des Blisters aus einem geeigneten Folien- oder anderen Material eingelegt und dieser auf der Seite der offenen Mündung der Aufnahmen durch eine Deckwand aus Karton, Kunststoff, Metall oder einem anderen geeigneten abreißbaren oder in anderer Weise zu öffnenden Material verschlossen ist.

Blisterverpackungen, in denen das abgepackte Gut in vorgeformten Aufnahmen eines Blisters aus einer steifen Kunststoff-Folie eingelegt und gegen Verlust durch eine auf die offene Mündungsseite des Blisters aufgeklebte, aufgesiegelte oder aufgeklammert Deckwand aus Karton, Folie o.dgl. gesichert gehalten ist, sind bekannt. Sofern das in die Aufnahmen der Blisterverpackung eingelegte Gut eine bestimmte äußere Form hat, werden die Aufnahmen im Blister jeweils etwa mit geringem Übermaß komplementär zum Umriß des aufzunehmenden Guts geformt, um seine bestimmungsgemäße Lage in der Blisterpackung zu stabilisieren und - bei stoßoder druckempfindlichem Gut - gegen Beschädigung bei unachtsamer Handhabung zu schützen. Dabei ist es auch möglich, in mehreren angepaßten Aufnahmen eines Blisters jeweils unterschiedliche, aber bei der späteren Anwendung gemeinsam erforderliche Komponenten anzuordnen und durch eine gemeinsame Deckwand gegen Verlust bis zur Anwendung zu schützen.

Aus der US-A- 4 844 251 ist eine Blister-Verpackungseinheit der eingangs erwähnten Art bekannt, wobei einer der in zugeordneten Aufnahmen des Blisters enthaltenen Komponenten eine pulver- oder granulatförmige Konsistenz hat.

Der Erfindung liegt die Aufgabe zugrunde, die bekannte Blister-Verpackungseinheit so zu verbessern, dass die Komponenten verlustfrei in eine Misch- oder Applikations-Vorrichtung eingebracht und zu einer gebrauchsfertigen Mischung aufbereitet werden können.

Ausgehend von einer Blister-Verpackungseinheit der eingangs erwähnten Art wird diese Aufgabe erfindungsgemäß dadurch gelöst, daß die unterschiedlichen Komponenten in gesonderten Einzelverpackungen voneinander getrennt abgepackt sind, von denen die die pulver- oder granulatförmige Konsistenz aufweisende(n) Komponente(n) jeweils in einer der zugeordneten Aufnahme des Blisters zumindest angenähert komplementär entsprechend geformten eigenen Blisterverpackung abgepackt ist bzw. sind, dessen bzw. deren Blister jeweils die Form eines langgestreckten, zumindest an einem Ende ausgußartige verjüngten Wannenelements aus Kunststoff hat, wobei das bzw. die Wannenelement(e) durch eine gesonderte abreißbare Deckwand aus Karton, Kunststoff, Metall oder einem anderen geeigneten abreißbaren oder in anderer Weise zu öffnenden Material verschlossen ist. Die pulver- oder granulatförmige Komponente kann beispielsweise eine vorgegebene Menge eines Polymers sein, welches vor Gebrauch mit einer vorgegebenen Menge eines flüssigen und dann zweckmäßig in einem Glasbehältnis in einer zweiten Aufnahme im Blister auf Vorrat gehaltenen flüssigen Monomers zu einem abbindenden oder aushärtendem Kleber oder Zement aufbereitet werden kann. Die Ausgestaltung des die pulver- oder granulatförmige Komponente aufnehmenden Blisters als Wanrienelement mit ausgußartig verjüngtem Ende stellt sicher, daß diese Komponente verlustfrei in die Misch- oder Applikations-Vorrichtung eingebracht werden kann, so daß das vorgegebene Mischungsverhältnis des fertig aufbereiteten Produkts gewährleistet ist.

Die erfindungsgemäße Blister-Verpackungseinheit bietet sich zur portionierten Abpackung der Bestandteile eines in der Humanmedizin, insbesondere der chirurgischen Orthopädie, anzuwendenden Knochenzements an, wobei das pulver- oder granulatförmige Polymer mit dem flüssigen Monomer unmittelbar vor der Anwendung zu einer pastenförmig zu verarbeitenden Masse in einem Applikator aufbereitet werden. Dabei ist die die pulver- oder granulatförmige Konsistenz aufweisende(n) Komponente(n), d.h. das Polymer dann in der zugeordneten Blisterverpackung steril abgepackt. Bei entsprechender Wahl des Materials der Deckwand der Blisterverpackung des Polymers kann diese Sterilisation auch nachträglich durch entsprechende Begasung oder alternativ durch Strahlensterilisation erfolgen. Dagegen stellt die sterile Abfüllung der flüssigen Komponente (Monomer) in einem Glasröhrchen etc. verfahrenstechnisch kein Problem dar.

Die Erfindung ist in der folgenden Beschreibung eines Ausführungsbeispiels in Verbindung mit der Zeichnung näher erläutert, und zwar zeigt:
- Fig. 1: eine Seitenansicht eines Ausführungsbeispiels einer erfindungsgemäßen Blister-Verpackungseinheit, welche zur sterilen und portionierten Aufbewahrung und Bereitstellung von zwei vor Gebrauch durch Vermischung in einer geeigneten Misch- und/oder Applikations-Vorrichtung bestimmten Komponenten bestimmt ist, von denen im speziellen Fall die eine eine pulveroder granulatförmige Konsistenz und die andere flüssige Konsistenz aufweist;
- Fig. 2: eine Unteransicht der Blister-Verpackungseinheit, gesehen in Richtung des Pfeils 2 in Fig. 1;
- Fig. 3: eine Ansicht der Blister-Verpackungseinheit, gesehen in Richtung des Pfeils 3 in Fig. 1;
- Fig. 4: eine Ansicht der Blister-Verpackungseinheit, gesehen in Richtung des Pfeils 4 in Fig. 1;
- Fig. 5: eine Draufsicht auf die Blister-Verpackungseinheit mit abgenommener Deckwand und ohne die eingelegten Einzelverpackungen für die beiden Komponenten, gesehen in Richtung des Pfeils 5 in Fig. 1;
- Fig. 6: eine Seitenansicht der gesonderten, die pulver- oder granulatförmige Komponente aufnehmenden Blisterverpackung;
- Fig. 7: eine Ansicht, gesehen in Richtung des Pfeils 7 in Fig. 6; und
- Fig. 8: eine Seitenansicht eines die zweite Komponente in der Blisterverpackung gemäß den Fig. 1 bis 5 aufzunehmenden Glasröhrchens.

Das in den Zeichnungen gezeigte Ausführungsbeispiel stellt eine in ihrer Gesamtheit mit 10 bezeichnete Blister Verpackungseinheit für die Komponenten eines unmittelbar vor der Anwendung durch Vermischung aufzubereitenden Zwei-Phasen-Knochenzementes dar, der aus einer pulverförmigen festen Phase aus Polymethylmetacrylat und einer flüssigen Phase aus monomerem Methylmetacrylat besteht, wobei der pulverförmigen festen Phase weitere Stoffe als Katalysator und der flüssigen Phase zusätzliche flüssige Bestandteile als Beschleuniger und Stabilisatoren bzw. weitere Zuschlagstoffe zugesetzt sein können. Die beiden Komponenten werden unmittelbar vor Gebrauch zu einem plastischen Produkt gemischt, welches im Laufe der Zeit aushärtet und z.B. in der orthopädischen Chirurgie zur Fixierung von Prothesen eingesetzt wird.

Die pulver- oder granulatförmige Polymer-Komponente wird - mitsamt eventuellen Zuschlagstoffen - in der vorgesehenen Menge in einem als langgestrecktes Wannenelement 12 ausgebildeten Blister eingefüllt, der durch eine auf einen an seinem freien Rand ausgebildeten ebenflächigen umlaufenden Flansch 14 aufgeklebte Deckwand aus Karton, Papier, Folie oder einem Papier-Folien-Laminat hermetisch dicht verschlossen wird. Durch geeignete Techniken, z.B. die Begasung des noch geöffneten oder mit einem gasdurchlässigen Material verschlossenen Wannenelements mit Äthylenoxid oder durch Einwirkung ionisierender Strahlung hinreichender Dosis bei gegebenenfalls bereits geschlossener Deckwand, wird die erforderliche Sterilität der Polymer-Komponenten gewährleistet.

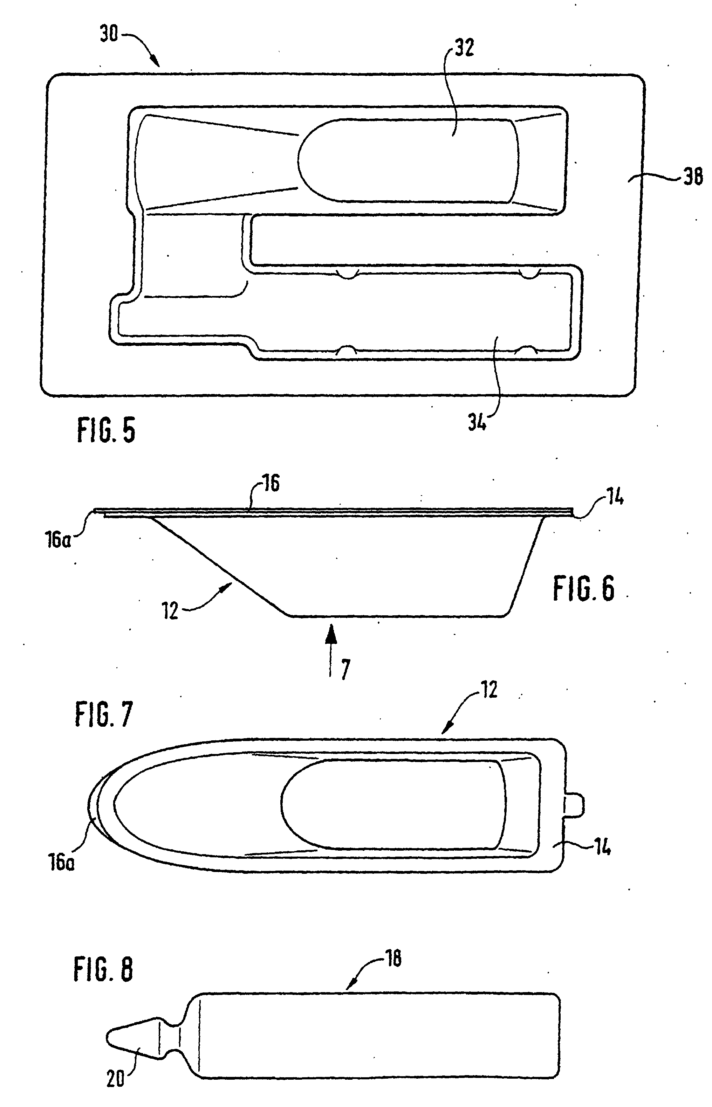

In den Fig. 6 und 7 ist erkennbar, daß das Wannenelement 12 an seinem in den Zeichnungsfiguren linken Ende sich ausgußartig und vom Boden schräg nach oben ansteigend verjüngt, so daß die abgepackte Komponente nach Abreißen der Deckwand 16 ohne Schwierigkeit und ohne Verschütten auch in einen engen Einguß eines Misch- und Applikationsgeräts einbringbar ist. Um das Öffnen, d.h. das Abreißen der Deckwand 16 vom Wannenelement 12 zu erleichtern, wird die Deckwand im vorderen verjüngten Ausgußbereich so vergrößert, daß sie eine Aufreißlasche 16a bildet.

Ein kleiner Einschnitt in dieser ermöglicht es, bei nur halb geöffnetem Wannenelement die Deckwand zum hinteren Ende hin zu klappen und auf die Vergrößerung im hinteren Ende des umlaufenden Flanschs 14 des Wannenelements (Fig. 7) einzuhängen. Dies erleichtert das Ausbringen des Inhalts und verhindert außerdem eine ungewollte Staubbildung beim Ausschütten feiner Pulver.

Die zweite flüssige oder Monomer-Komponente wird in der für die Mischung die optimalen angestrebten Eigenschaften erzielenden Menge steril in einem langgestreckten zylindrischen Glasröhrchen 18 mit integral angesetzter abbrechbarer Ausgußtülle 20 abgefüllt, die nach der Befüllung mit dem Monomer durch Verschmelzung ihrer offenen Mündung hermetisch dicht verschlossen ist.

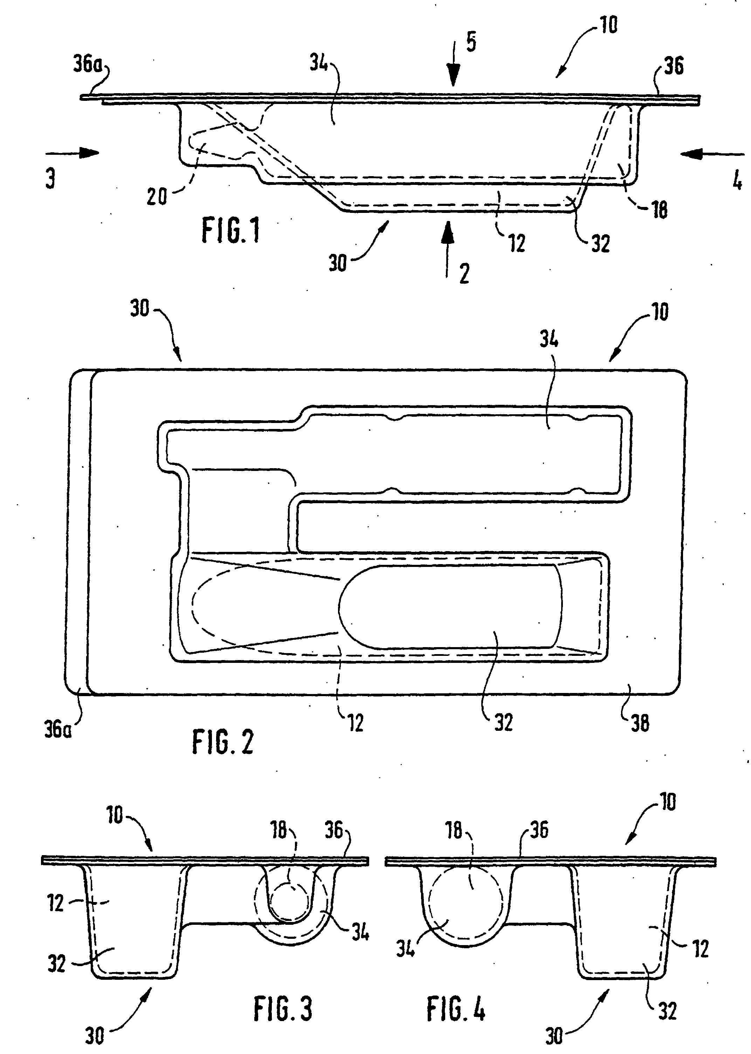

Die bis zum Gebrauch unverlierbare und gegen Beschädigung gesicherte Verpackung der beiden Komponenten erfolgt in der erwähnten Blister-Verpackungseinheit 10, die in den Fig. 1 bis 4 dargestellt ist und sich aus dem eigentlichen Blister 30 (Fig. 5) aus Kunststoff-Folie, in welchem den Einzelverpackungen der Komponenten etwa entsprechende Aufnahmen 32, 34 eingeformt sind, und einer Deckwand 36 aus Karton, Papier, Folien oder einem Papier-Folien-Laminat zusammensetzt, die auf der Seite der offenen Mündung der Aufnahmen 32, 34 ausgebildeten ebenflächigen Randflansch 38 aufgeklebt bzw. aufgesiegelt ist. Die Deckwand 36 kann - zumindest an einer Begrenzungsseite den Randflansch 36 in der in Fig. 2 am linken Rand gezeigten Weise etwas überragen und bildet dort dann eine Aufreißlasche 36a.

Die Blister-Verpackungseinheit 10 ist so gestaltet, daß der umlaufende Flansch nacheinander mit zwei verschiedenen Materialien verschlossen werden kann. So ist es möglich, in einem ersten Schritt nach dem Verschluß mit einem gasdurchlässigen Deckel beispielsweise eine Äthylenoxidbehandlung zum Zwecke einer Sterilisation des Blisterinhalts durchzuführen. In einem zweiten Schritt kann dann durch Anbringen eines luft- und wasserdampfdichten Deckels, beispielsweise aus einer aluminiumkaschierten Folie, eine hermetisch dichte Versiegelung erfolgen. Natürlich kann auch eine wasserdampfdichte Verpackung dadurch erreicht werden, daß der gesamte Blister mit Inhalt nach seinem Verschluß in eine, beispielsweise aluminiumkaschierte, Folie hermetisch eingeschweißt wird.

Es ist ersichtlich, daß im Rahmen des Erfindungsgedankens Abwandlungen und Weiterbildungen des beschriebenen Ausführungsbeispiels verwirklichbar sind, die sich beispielsweise auf die Anzahl der gemeinsam jeweils in gesonderten Einzelverpackungen in der erfindungsgemäßen Blister-Verpackungseinheit abgepackt zu bevorratenden Komponenten bezieht. So ist es beispielsweise denkbar, zwei (oder mehr) pulveroder granulatförmige Komponenten jeweils in gesonderten, der Blisterpackung 12, 14, 16 ähnlichen Packungen in der gemeinsamen Blister-Verpackungseinheit 10 zu bevorraten, die dann eine zusätzliche Aufnahme im Blister 30 aufweisen muß. Eine solche Blister-Verpackungseinheit ist beispielsweise dann zweckmäßig, wenn - im Kontext mit dem Anwendungsfall des vorstehend beschriebenen Ausführungsbeispiels - das aufzubereitende Produkt zusätzlich eine weitere, beispielsweise pharmazeutisch wirksame Komponente, z.B. wie ein Antibiotikum etc., enthalten soll, welches in der Mischung weitgehend homogen verteilt sein muß. Sofern die zusätzliche Komponente flüssige Kosistenz hat, wird sie in der Regel mit dem flüssigen Polymer vormischbar sein. In pulvriger oder kristalliner Form kann es jedoch zweckmäßig sein, diese pharmazeutisch wirksame Komponente zunächst in hoher Dosierung mit einer Teilmenge der pulver- oder granulatförmige Kosistenz aufweisenden Komponente, d.h. der Polymer-Komponente, zu mischen und den der fertigen Mischung enthaltenen Anteil durch Zugabe der ohne die pharmazeutisch wirksame Substanz gesondert auf Vorrat gehaltenen pulver- oder granulatförmigen Komponenten einzustellen. Auch auf die Dauer unverträgliche flüssige Komponenten können jeweils zunächst in gesonderten Einzelverpackungen in zugeordneten Aufnahmen in der erfindungsgemäßen Blisterverpackung bevorratet sein und werden dann erst unmittelbar vor der Anwendung miteinander vermischt.

## Patentansprüche

1. Blister-Verpackungseinheit (10), in welcher wenigstens zwei unterschiedliche Komponenten unverlierbar enthalten sind, von denen wenigstens eine eine pulver- oder granulatförmige Konsistenz hat, die vor Gebrauch mit der bzw. den weiter abgepackten Komponente(n) in einer Misch- oder Applikations-Vorrichtung zu einer gebrauchsfertigen Mischung aufbereitet werden sollen, wobei die Komponenten in entsprechend vorgeformten Aufnahmen (32; 34) des Blisters (30) aus einem geeigneten Folien- oder anderen Material eingelegt und dieser auf der Seite der offenen Mündung der Aufnahmen durch eine Deckwand (36) aus Karton, Kunststoff, Metall oder einem anderen geeigneten abreißbaren oder in anderer Weise zu öffnenden Material verschlossen ist,
**dadurch gekennzeichnet,**
**daß** die unterschiedlichen Komponenten in gesonderten Einzelverpackungen voneinander getrennt abgepackt sind, von denen die die pulver- oder granulatförmige Konsistenz aufweisende(n) Komponente(n) jeweils in einer der zugeordneten Aufnahme (32) des Blisters zumindest angenähert komplementär entsprechend geformten eigenen Blisterverpackung (12; 14; 16) abgepackt ist bzw. sind, dessen bzw. deren Blister jeweils die Form eines langgestreckten, zumindest an einem Ende ausgußartig verjüngten Wannenelements (12) aus Kunststoff hat, wobei das bzw. die Wannenelement(e) durch eine gesonderte abreißbare Deckwand (16) aus Karton, Kunststoff, Metall oder einem anderen geeigneten abreißbaren oder in anderer Weise zu öffnenden Material verschlossen ist.

2. Blister-Verpackungseinheit nach Anspruch 1, bei dem die pulver- oder granulatförmige Konsistenz aufweisende(n) Komponente(n) steril abgepackt ist bzw. sind und die Sterilität bis zur Aufbereitung zur gebrauchsfertigen Mischung gewährleistet bleiben muß, **dadurch gekennzeichnet, daß** sowohl das Material des Wannenelements (12) als auch das Material der gesonderten Deckwand (16) und deren Verbindung gegen den Durchtritt von Keimen undurchlässig ausgebildet sind.

3. Blister-Verpackungseinheit nach Anspruch 2, **dadurch gekennzeichnet, daß** das Material des Wannenelements (12) und/oder der gesonderten Deckwand (16) für die Durchstrahlung mit ionisierender Strahlen zum Zweck der Strahlen-Sterilisation der pulverförmige oder granulatförmige Konsistenz aufweisende(n) Komponente(n) durchlässig ausgebildet ist.

4. Blister-Verpackungseinheit nach einem der Ansprüche 1 bis 3, **dadurch gekennzeichnet, daß** die abreißbare gesonderte Deckwand (16) für die Begasung beispielsweise mit Äthylenoxid zum Zwecke der Sterilisation der pulverförmige oder granulatförmige Konsistenz aufweisende(n) Komponente(n) durchlässig ausgebildet ist.

5. Blister-Verpackungseinheit nach einem der Ansprüche 1 bis 3, **dadurch gekennzeichnet, daß** auf dem umlaufenden Flansch (14) des Wannenelements (12) in zwei aufeinanderfolgenden Schritten zwei gesonderte Deckwände (16) mit unterschiedlichen chemisch-physikalischen Eigenschaften aufgebracht sind.

## Claims

1. Blister packaging unit (10) in which at least two different components are retained captive, at least one of which has a pulverulent or granular consistency and is to be prepared before use with the further packaged component(s) in a mixer or applicator to form a ready-for-use mixture, whereby the components are inserted in appropriately preshaped receptacles (32; 34) in the blister (30) of a suitable film or other material and the latter is closed on the side of the open mouth of the receptacles by a cover (36) of cardboard, plastic, metal or some other suitable material which may be torn off or opened in some other manner, **characterised in that** the different components are packed separated from one another in separate individual packages, of which the component(s) having the pulverulent or granular consistency is/are packaged in an individual blister package (12; 14;16) which is shaped at least approximately complementarily to the associated receptacle (32) in the blister, the blister of which has the shape of an elongate trough element (12) of plastic tapered in the manner of a spout at at least one end, whereby the trough element(s) is closed by a separate tear-off cover (16) of cardboard, plastic, metal or some other suitable material which may be torn off or opened in some other manner.

2. Blister packaging unit as claimed in Claim 1, in which the component(s) having a pulverulent or granular consistency is/are packaged in a sterile manner and the sterility must be guaranteed until preparation to form the ready-to-use mixture, **characterised in that** both the material of the trough element (12) and the material of the separate cover (16) and their connection is constructed to be impermeable to the passage of germs.

3. Blister packaging unit as claimed in Claim 2, **characterised in that** the material of the trough element (12) and/or the separate cover (16) is constructed to be permeable to radiation with ionising beams for the purpose of radiation sterilisation of the component(s) having a pulverulent or granular consistency.

4. Blister packaging unit as claimed in one of Claims 1 to 3, **characterised in that** the separate tear-off cover (16) is constructed to be permeable for gas treatment, for instance with ethylene oxide, for the purpose of sterilising the component(s) having a pulverulent or granular consistency.

5. Blister packaging unit as claimed in one of Claims 1 to 3, **characterised in that** two separate covers (16) with different chemical, physical properties are applied in two successive steps to the peripheral flange (14) of the trough element (12).

## Revendications

1. Unité d'emballage coque (10) contenant de manière imperdable au moins deux constituants différents dont au moins une a une consistance de poudre ou de granulé, qui sont destinés, avant emploi, à être préparés avec le ou les autres constituants emballés, dans un appareil de mélange ou d'application, en un mélange prêt à l'emploi, les constituants étant placés dans des cases de forme appropriée (32 ; 34) de 1a coque (30) constituée d'une matière en feuille ou autre appropriée, et la coque étant fermée sur le côté de l'orifice ouvert des cases par une paroi de couverture (36) en carton, plastique, métal ou une autre matière arrachable, ou s'ouvrant d'une autre manière, appropriée,
**caractérisée par le fait**
**que** les différents constituants sont emballés séparément dans des emballages individuels séparés, dont le ou chaque constituant présentant la consistance de poudre ou de granulé est emballé dans un emballage coque propre (12; 14; 16) de forme au moins approximativement complémentaire de celle de la case associée (32) de la coque, constituant dont la coque a la forme d'un élément cuvette allongé (12) rétréci à la manière d'un bec au moins à une extrémité, l'élément cuvette étant fermé par une paroi de couverture arrachable séparée (16) en carton, plastique, métal ou une autre matière arrachable, ou s'ouvrant d'une autre manière, appropriée.

2. Unité d'emballage coque selon la revendication 1, dans laquelle le ou chaque constituant présentant une consistance de poudre ou de granulé est emballé de manière stérile et la stérilité doit rester assurée jusqu'à la préparation du mélange prêt à l'emploi, **caractérisée par le fait que** la matière de l'élément cuvette (12) et la matière de la paroi de couverture séparée (16) et leur jonction sont imperméables au passage de germes.

3. Unité d'emballage coque selon la revendication 2, **caractérisée par le fait que** la matière de l'élément cuvette (12) et/ou de la paroi de couverture séparée (16) est perméable au passage de rayonnements ionisants pour la stérilisation par rayonnement du constituant ou des constituants présentant une consistance de poudre ou de granulé.

4. Unité d'emballage coque selon l'une des revendications 1 à 3, **caractérisée par** le fait sur la paroi de couverture séparée arrachable (16) est perméable aux gaz, par exemple à l'oxyde d'éthylène pour la stérilisation du constituant ou des constituants présentant une consistance de poudre ou de granulé.

5. Unité d'emballage coque selon l'une des revendications 1 à 3, **caractérisée par le fait que** sur le rebord périphérique (14) de l'élément cuvette (12) sont appliquées en deux étapes successives deux parois de couverture séparées (16) ayant des propriétés physicochimiques différentes.
